# EUROPEAN PATENT SPECIFICATION

(11) **EP 3 542 709 B1**
(45) Date of publication and mention of the grant of the patent: **24.12.2025**
(21) Application number: 18163613.5
(22) Date of filing: 23.03.2018
(51) Int. Cl.: A61B 5/00, H01R 13/62, A61B 5/024

(54) **AN APPARATUS AND WEARABLE ELECTRONIC DEVICE FOR SENSING**
VORRICHTUNG UND ELEKTRONISCHE WEARABLE-VORRICHTUNG ZUR ERFASSUNG
APPAREIL ET DISPOSITIF ÉLECTRONIQUE PORTABLE DE DÉTECTION

(43) Date of publication of application: 25.09.2019
(73) Proprietor: Nokia Technologies Oy, 02610 Espoo (FI)
(72) Inventor: RADIVOJEVIC, Zoran, Cambridge, Cambridgeshire CB3 0FA (GB)
(74) Representative: Swindell & Pearson Limited

(56) References cited:
- EP-A1- 3 225 156
- WO-A1-2016/151433
- US-A1- 2011 066 049
- US-A1- 2016 287 103
- US-A1- 2017 222 359

## Description

### TECHNOLOGICAL FIELD

Examples of the disclosure relate to an apparatus and wearable electronic device for sensing. The apparatus and wearable device may be configured to sense one or more biometric parameters of a subject.

### BACKGROUND

Apparatus for detecting biometric parameters are known. For example, sensors configured to detect parameters such as heart rate can be integrated within wearable devices such as chest straps or watches.

US 2011/066049 discloses a device for detecting pulse abnormality. The device comprises a band attached to a wrist, on which a pulse sensor is provided. The position of the sensor may be adjusted.

US 2017/222359 discloses a smart contact device. The device comprises an attachment face and a contact array comprising one or more magnets.

WO 2016/151433 discloses a band for positioning a medical monitoring device on a medical subject. The band is secured around the subject by a magnetic coupling of at least one magnet and at least one ferromagnetic portion.

EP 3 225 156 discloses adaptive wearable devices that measure physiological conditions. The devices comprise a support structure that encloses the torso or an appendage of a user, and a sensor positioned on a spring module.

### BRIEF SUMMARY

The invention is defined by the claims.

According to various, but not necessarily all, examples of the disclosure there is provided an apparatus comprising: a sensor module comprising one or more sensors wherein the one or more sensors are configured to detect one or more biometric parameters of a subject; means for attaching the apparatus to a subject; and means for adjusting the position of the sensor module relative to the means for attaching the apparatus to a subject such that when the apparatus is attached to a subject the sensor module can move relative to the means for attaching the apparatus to a subject, but movement of the sensor module relative to the subject is restricted, the means for adjusting comprising: a first magnetic portion, provided within the sensor module; and a second magnetic portion, provided on the means for attaching the apparatus to a subject.

The apparatus may be configured such that when the apparatus is attached to a subject by the means for attaching the apparatus to a subject, the one or more sensors are positioned adjacent to the subject.

The one or more sensors may be provided within a sensor module. The sensor module may comprise one or more pads on a surface of the sensor module wherein the pads are positioned such that when the apparatus is attached to a subject the one or more pads contact the subject. The one or more pads may comprise elastomeric material.

The means for adjusting may comprise an array of magnets arranged in a repelling configuration.

The means for adjusting may comprise one or more conductive portions.

The means for adjusting may be configured to enable the one or more sensors to move in a longitudinal direction relative to the means for attaching the apparatus to a subject.

The means for adjusting may be configured to enable the one or more sensors to rotate relative to the means for attaching the apparatus to a subject.

The means for attaching the apparatus to a subject may comprise a strap.

The one or more sensors may comprise one or more optical sensors.

According to various, but not necessarily all, examples of the disclosure there may be provided wearable electronic device comprising an apparatus as claimed in any preceding claim and controlling circuitry.

The wearable electronic device may comprise one or more transceivers.

According to various, but not necessarily all, examples of the disclosure there is provided an apparatus comprising: one or more sensors wherein the one or more sensors are configured to detect one or more biometric parameters of a subject; at least one attachment portion configured to attach the apparatus to a subject; and at least one adjustment portion configured to adjust the position of the one or more sensors relative to the attachment portion such that when the apparatus is attached to a subject the one or more sensors can move relative to the attachment portion.

### BRIEF DESCRIPTION

For a better understanding of various examples that are useful for understanding the detailed description, reference will now be made by way of example only to the accompanying drawings in which:
Fig. 1 illustrates an example apparatus;
Fig. 2 illustrates an example apparatus;
Figs. 3A to 3D are photographs of an example apparatus;
Figs. 4A to 4E are photographs of an example apparatus;
Figs. 5A to 5D illustrates another example apparatus; and
Fig. 6 illustrates a wearable device comprising an example apparatus.

### DETAILED DESCRIPTION

Examples of the disclosure relate to an apparatus 101 which could be integrated within a wearable device 601. The wearable device 601 could be worn by a subject to enable one or more biometric parameters of the subject to be sensed. The apparatus 101 is configured so that when the apparatus 101 is in use one or more sensors 103 comprised within the apparatus 101 can move relative to a strap or other attachment means but the movement of the sensors 103 relative to the subject is restricted. This enables accurate and reliable measurements of the biometric parameters to be made while providing for the comfort of the subject.

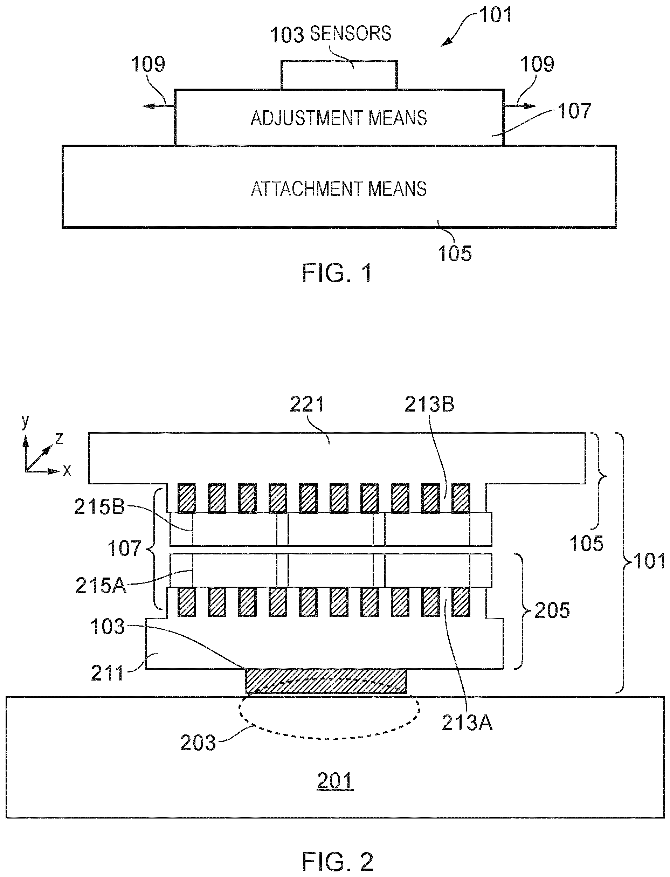

Fig. 1 schematically illustrates an apparatus 101 according to examples of the disclosure. The apparatus 101 comprises one or more sensors 103, attachment means 105 for attaching the apparatus 101 to a subject and adjustment means 107 for adjusting the position of the one or more sensors 103.

The one or more sensors 103 may provide sensing means. The sensors 103 may comprise any means which may be configured to sense one or more parameters. The sensors 103 may be configured to provide an electrical output signal dependent upon the one or more parameters.

The parameters that are sensed by the one or more sensors 103 comprise a biometric parameter of a subject. The biometric parameter may comprise any time varying signal that is generated by the subject's body. The biometric parameter may comprise an autonomic parameter. The autonomic parameter may be controlled subconsciously by the subject. The biometric parameters could comprise heart rate, temperature, humidity, blood oxygen levels, audio signals, pressure, presence of one or more analytes or any other suitable parameters.

The one or more sensors 103 may be positioned within the apparatus 101 to enable the sensors 103 to detect the biometric parameters. The sensors 103 may be positioned within the apparatus 101 so that when the apparatus 101 is in use the sensors 103 are positioned adjacent to the subject. In some examples the apparatus 101 may be configured so that when the attachment means 105 is attached to a subject the sensors 103 are in contact with the subject. The apparatus 101 may be configured so that when the attachment means 105 is attached to a subject the sensors 103 are in direct contact with the subject.

The one or more sensors 103 are coupled to the attachment means 105. The attachment means 105 provide means for attaching the apparatus 101 to a subject. In some examples the attachment means 105 may comprise a strap or other flexible member. The attachment means 105 may be sized and shaped so as to enable it to be wrapped around part of a subject's body. The attachment means 105 may be sized and shaped to fit around a subject's arm or around a subject's torso or around any other suitable part of the subject's body.

The attachment means 105 may comprise any suitable material. In some examples the attachment means 105 may comprise a flexible material which is flexible enough to enable the attachment means 105 to be deformed around a part of the subject's body. In some examples the attachment means 105 may comprise textile, polymer, leather or any other suitable material or combination of materials. In some examples the attachment means 105 could comprise a stretchable or elastic material or a chained structure made from a non-elastic material such as metal, or any other suitable flexible structure. In some examples the attachment means 105 may comprise a plurality of different materials. The different materials may be provided in different layers within the attachment means 105.

In examples of the disclosure the one or more sensors 103 are coupled to the attachment means 105 via the adjustment means 107. The adjustment means 107 provide means for adjusting the position of the one or more sensors 103 relative to the attachment means 105. The adjustment means 107 enable the one or more sensors 103 to move relative to the attachment means 105. In some examples the adjustment means 107 may enable the one or more sensors 103 to move relative to the attachment means 105 while the apparatus 101 is attached to a subject. The adjustment means 107 restrict the movement of the sensors 103 relative to the subject. This may enable the sensors 103 to be fixed in position relative to the subject so as to provide consistent sensing measurements but allows the position of the sensors 103 to be adjusted relative to the attachment means 103 and/or other components of the apparatus 101 which make the apparatus 101 more comfortable to use.

The adjustment means 107 may comprise any suitable means for enabling movement of the one or more sensors 103. The adjustment means 107 comprise magnetic portions which may enable the position of the sensors 103 to be adjusted while maintaining an electrical connection between the sensors 103 and the rest of the apparatus 101. The means 107 comprise one or more magnetic portions which may enable the position of the sensors 103 to be adjusted while maintaining a good electrical connection between the sensors 103 and the rest of the apparatus 101. The good electrical connection may be a connection which is sufficient for signals to be transmitted between the sensors 103 and the rest of the apparatus 101.

In the example of Fig. 1 arrows 109 indicate the directions in which the sensors 103 can be moved via the adjustment means 107. In the example of Fig. 1 the arrows 109 indicate that the sensors 103 can move laterally along a given direction. The direction may correspond to a length of the attachment means 105. The adjustment means 107 may be configured so that the sensors 103 can move forwards and/or backwards along the direction indicated by the arrows 109. It is to be appreciated that other types of movement may be enabled in other examples of the disclosure.

For instance, in some examples of the disclosure the adjustment means 107 may be configured to enable the sensors 103 to be rotated relative to the attachment means 105.

Fig. 2 illustrates a cross section of an apparatus 101 according to examples of the disclosure. The apparatus 101 comprises a sensor 103, attachment means 105 and adjustment means 107. It is to be appreciated that the apparatus 101 could comprise additional components that are not shown in Fig. 2. For example, the apparatus 101 could comprise more than one sensor 103.

In the example of Fig. 2 the apparatus 101 is attached to a subject. The apparatus 101 is positioned so that a surface of the sensor 103 is in contact with the skin 201 of the subject. This provides an interface between the sensor 103 and the skin 201 of the subject. This enables the sensor 103 to detect biometric parameters from the subject.

In the example of Fig. 2 the sensor 103 may comprise an optical sensor. The optical sensor may be configured to provide light to the surface of the subject's skin 201 and to reflect light from the surface of the skin 201. This may enable information about biometric parameters of the subject to be obtained. For example it may enable a heart rate, or blood oxygenation level or other suitable parameter to be monitored. It is to be appreciated that other types of sensor 103 could be used in other examples of the disclosure.

The sensor 103 is provided within a sensor module 205. The sensor module 205 comprises a substrate 211. The one or more sensors 103 are mounted on a first surface of the substrate 211. The substrate 211 may be configured to bear the weight, or at least partially, bear the weight of the sensors 103. The substrate 211 may also comprise additional components such as electrical connections which may enable the sensor 103 to be electrically connected to other components of the apparatus 101.

The substrate 211 may comprise any suitable material. The substrate 211 could be a flexible substrate or a rigid substrate. In some examples the substrate 211 may comprise silicon.

In the example of Fig. 2 the substrate 211 also comprises part of the adjustment means 107. The parts of the adjustment means 107 that are provided on the substrate 211 comprise a first magnetic portion 213A and a first conductive portion 215A. The first magnetic portion 213A and the first conductive portion 215A are provided on an opposing surface of the substrate 211 to the sensors 103.

The first magnetic portion 213A may comprise any suitable magnetic material. In the example apparatus 101 of Fig. 2 the first magnetic portion 213A is configured to provide a magnetic layer between the substrate 211 and the attachment means 105. In the example of Fig. 2 the first magnetic portion 213A provides a magnetic layer between the substrate 211 and the first conductive portion 215A.

In the example of Fig. 2 the magnetic portion 213 is provided on a surface of the substrate 211. In other examples the magnetic portion 213 could be provided in a different position. For example the magnetic portion 213 could be embedded within the substrate 211 or at some other location within the sensor module 205.

In some examples the first magnetic portion 213A may comprise a magnetized material which provides a magnetic field. In some examples the first magnetic portion 213A may comprise a material that is responsive to the magnetic field generated by another magnetic portion. The first magnetic portion 213A may comprise magnetic rare earth metals such as neodymium, alloys comprising rare earth metals or any other suitable magnetic material.

In the example of Fig. 2 the first magnetic portion 213A comprises a plurality of magnetic blocks 219. The plurality of magnetic blocks 219 may comprise individual magnets. A gap may be provided between adjacent magnetic blocks 219.

In the example of Fig. 2 the magnetic blocks 219 may be arranged in a linear array. The linear array may comprise a single row of magnetic blocks 219. It is to be appreciated that in other examples the plurality of magnetic blocks 219 could be arranged in arrays having a plurality of rows and columns or on any other suitable configuration.

The magnetic blocks 219 may be arranged in a repelling configuration. In the repelling configuration the magnetic blocks 219 are positioned within the array so that adjacent magnetic blocks 219 have repelling polarities. For example, each of the magnetic blocks 219 has a north pole and a south pole. The north and south poles are provided at the edges of the magnetic blocks 219. The magnetic blocks 219 are provided in alternating directions so that the north pole of a magnetic block 219 is adjacent to the north pole of a neighbouring magnetic block 219 and the south pole of the magnetic block 219 is adjacent to the south pole of a neighbouring magnetic block 219.

The repelling configuration provides for a magnetic field which has a large gradient in the region between two adjacent magnetic blocks 219. This may provide a large magnetic force on a magnet positioned in the region between two adjacent magnetic blocks 219. This may help to keep the first magnetic portion 213A aligned with a corresponding second magnetic portion 213B which may be provided on the attachment means 105.

In the example of Fig. 2 the first conductive portion 215A is also provided on the substrate 211. In the example of Fig. 2 the first conductive portion 215A is provided overlaying the first magnetic portion 213A. The first conductive portion 215A may be provided on the substrate 211 so that the first conductive portion 215A can be in contact with other conductive portions of the apparatus 101. For example the first conductive portion 215A may be in contact with a corresponding second conductive portion 215B provided on the attachment means 105. The contact between the first conductive portion 215A and the second conductive portion 215B may enable the sensor 103 to be connected to other components of the apparatus 101. For example it may enable the sensor 103 to be connected to circuitry which may be provided on the attachment means 105.

In the example of Fig. 2 the first conductive portion 215A comprises three conductive members. The three conductive members are spaced laterally along the surface of the substrate 211 of the sensor module 205. The three conductive members are spaced in the x direction as indicated in the axis shown in Fig. 2. The three conductive members may extend into the page towards the z direction. The conductive members may be elongate members so that the length of the members in the z direction is greater than the width of the members in the x direction. The length of the members in the x direction may be several times larger, or many times larger, than the width of the members in the x direction.

The parts of the adjustment means 107 that are provided on the sensor module 205 may be coupled to corresponding parts of the adjustment means 107 which may be provided on the attachment means 105. In the example of Fig. 2 the attachment means 105 may comprise a flexible substrate 221. The flexible substrate 221 may be flexible so that the attachment means 105 can be wrapped around a limb or torso or other part of the body of a subject. In the example apparatus 101 of Fig. 2 the parts of the adjustment means 107 that are provided on the attachment means 105 comprises a second magnetic portion 213B and a second conductive portion 215B.

The second magnetic portion 213B may have the same, or a similar, configuration to the first magnetic portion 213A. In the example of Fig. 2 the second magnetic portion 213B also comprises an array of magnetic blocks 219 arranged in a repelling configuration.

The second magnetic portion 213B is positioned within the apparatus 101 so that it can be aligned with the first magnetic portion 213A. The alignment of the two magnetic portions 213A and 213B provides an overlap between at least part of the first magnetic portion 213A and at least part of the second magnetic portion 213B. The magnetic fields of the respective portions 213A, 213B provide a restoring force to maintain the respective portions 213A 213B in the overlapping configuration. This therefore restricts lateral motion of the sensor module 205 relative to the attachment means 105. In the example of Fig. 2 the magnetic portions 213A, 213B restrict motion of the sensor module 105 in the x direction as indicated by the axis on Fig. 2. In other examples of the disclosure the magnetic portions 213A, 213B could be configured to restrict motion in different directions.

The second conductive portion 215B has a corresponding configuration to the first conductive portion 213A. In the example of Fig. 2 the second conductive portion 215B also comprises three conductive members where the three conductive members are spaced laterally along the surface of the attachment means 105. The conductive members of the second conductive portion 215B may be sized and shaped so that when the sensor module 205 is coupled to the attachment means 105 the conductive members of the first conductive portion 215A contact the conductive members in the second conductive portion 215B. In the example of Fig. 2 each of the conductive members of the first conductive portion 215A contact a conductive member in the second conductive portion 215B.

The contact between the conductive members of the first conductive portion 215A and the conductive members of the second conductive portion 215B provides a direct current connection between the first conductive portion 215A and the second conductive portion 215B. This therefore provides a direct current connection between the sensor 103 and any other components, such as circuitry or transceivers, which may be provided on the attachment means 105. The direct current connections may comprise a galvanic connection between the sensors 103 and other components of the apparatus 101. It is to be appreciated that both alternating current signals and direct current signals can be transmitted via the direct current path.

In the example of Fig. 2 the conductive portions 215A, 215B and the magnetic portions 213A, 213B are provided as separate components. In the example of Fig. 2 the conductive portions 215A, 215B are provided overlaying the respective magnetic portions 213A, 213B. In other examples the respective magnetic portions 213A, 213B and the respective conductive portions 215A, 215B could be provided as single components. For example the conductive portions 215A, 215B could be formed from a material which is both magnetic and conductive such as iron.

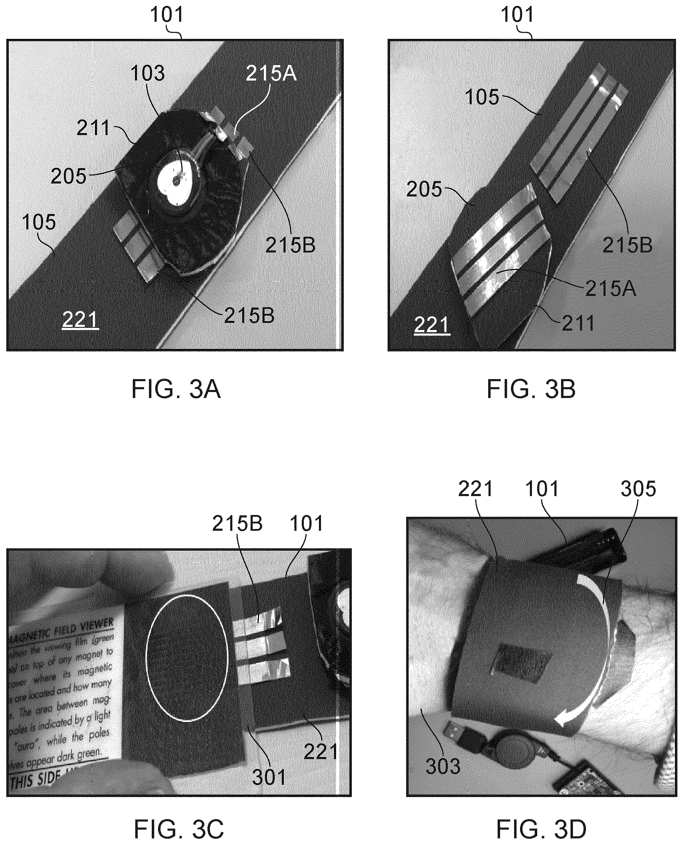

Figs. 3A to 3D are photographs of an example apparatus 101. The apparatus 101 shown in the photographs could be the same as the apparatus 101 shown in Fig. 2. The apparatus 101 comprises attachment means 105, a sensor 103 provided within a sensor module 205 and adjustment means.

Fig. 3A shows the sensor module 205 coupled to the attachment means 105. The sensor module 205 comprises a substrate 211 with an optical sensor 103 provided in a central position on a surface of the substrate 211. In other examples of the disclosure the sensor 103 could be provided in a different position on the surface of the substrate 211. In some examples the apparatus 101 could comprise more than one sensor 103 provided on the surface of the substrate 211.

In the example of Figs. 3A to 3D the sensor 103 may comprise an optical sensor. Other types of sensor may be used in other examples of the disclosure.

The first conductive portion 215A is provided on the reverse surface of the sensor module 205. The first conductive portion 215A comprises three conductive members. The ends of the conductive members can be seen in Fig. 3A.

The attachment means 105 comprises a flexible substrate 221. The second conductive portion 215B is provided on the inner surface of the flexible substrate 221. The second conductive portion 215B also comprises three conductive members. The three conductive members extend along a length of the flexible substrate 221. The three conductive members extend for a length which is greater than the length of the sensor module 205. This means that when the sensor module 205 is placed in a central position of the conductive members the ends of the conductive members project out from underneath the substrate 211 of the sensor module 205. This enables the sensor module 205 to be positioned anywhere along the length of the conductive members while still maintaining the electrical connection between the sensor module 205 and the attachment means 105. The respective conductive members therefore enable the position of the sensor module 205 to be adjusted.

Fig. 3B shows the sensor module 205 removed from the attachment means 105 so that the respective conductive portions 215A, 215B can be seen more clearly.

In the example apparatus 101 the first conductive portion 215A comprises three conductive members which extend across the rear surface of the substrate 211 of the sensor module 205. In the example of Fig. 3B the conductive members extend across the whole length of the rear surface of the substrate 211. In other examples the conductive members might extend only partway across the rear surface of the substrate 211.

The second conductive portion 215B comprises three corresponding conductive members which extend across the inner surface of the substrate 221 of the attachment means 105. In the example of Fig. 3B the conductive members extend across a part of the length of the inner surface of the substrate 221. The conductive members in the second conductive portion 215B may have a corresponding configuration to the conductive members in the first conductive portion 215A. For example the size and spacing of the conductive members in the second conductive portion 215B may be the same as the size and spacing of the conductive members in the first conductive portion 215A so that each of the conductive members in the first conductive portion 215A can contact a conductive member in the second conductive portion 215B.

In the example of Fig. 3B each of the conductive members has the same, or substantially the same width. The conductive members extend parallel, or substantially parallel, to each other. Other configurations for the conductive members may be used in other examples of the disclosure.

In the example of Fig. 3B the conductive members in the second conductive portion 215B have a longer length than the conductive members in the first conductive portion. This enables the sensor module 205 to be positioned at different locations on the attachment means 105 and so enables the position of the sensor module 205 to be adjusted.

Fig. 3C shows a magnetic field viewer 301 positioned over the apparatus 101 to show the second magnetic portion 213B. In the example apparatus 101 the second magnetic portion 213A is embedded in flexible substrate 221 of the attachment means 105 but can be seen through the magnetic field viewer 301. This shows the magnetic blocks 219 configured in a repelling arrangement.

In Fig. 3C the sensor module 205 has been removed from the attachment means 105 so that the magnetic portion 213B can be shown. It is to be appreciated that a corresponding first magnetic portion 213A may be provided in the sensor module 205.

The respective magnetic portions 213A, 213B act to restrict movement of the sensor module 205 across the width of the attachment means 105. However the configuration of the magnetic fields is such that they do not restrict the movements of the sensor module 205 along the length of the conductive members. The magnetic portions 213A, 213B therefore ensure that a good electrical connection is maintained between the sensor module 205 and the attachment means 105.

Fig. 3D shows the apparatus 101 in use attached to a subject's wrist 303. When the apparatus 101 is attached to the subject's wrist 303 the sensor 103 is positioned adjacent to the skin 201 of the subject and the attachment means 105 is fastened around the subject's wrist 303. The inner surface of the attachment means 105 may also be in contact, or partially in contact, with the subject's skin 201.

Once the apparatus 101 is attached to the subject the attachment means 105 could move relative to the subject's wrist 303 as indicated by the arrow 305. This movement could be caused by the movement of the subject. For example as the subject is walking, or otherwise moving, this could cause movement of the attachment means 105. This movement could be unintentional movement. In some examples the movement could be intentional, for example the movement could be the user adjusting the position of the attachment means 105 to a more comfortable position.

However, although the attachment means 105 may be moved the sensor module 205 may remain in a fixed position relative to the subject. The adjustment means 107 enables the sensor module 205 to move relative to the attachment means 105 and so enables the sensor module 205 to remain in a fixed position even when the attachment means 105 has moved. The friction between the surface of the sensor module 205 and the surface of the subject's skin 201 may be sufficient to prevent the sensor module from moving when the attachment means 105 moves.

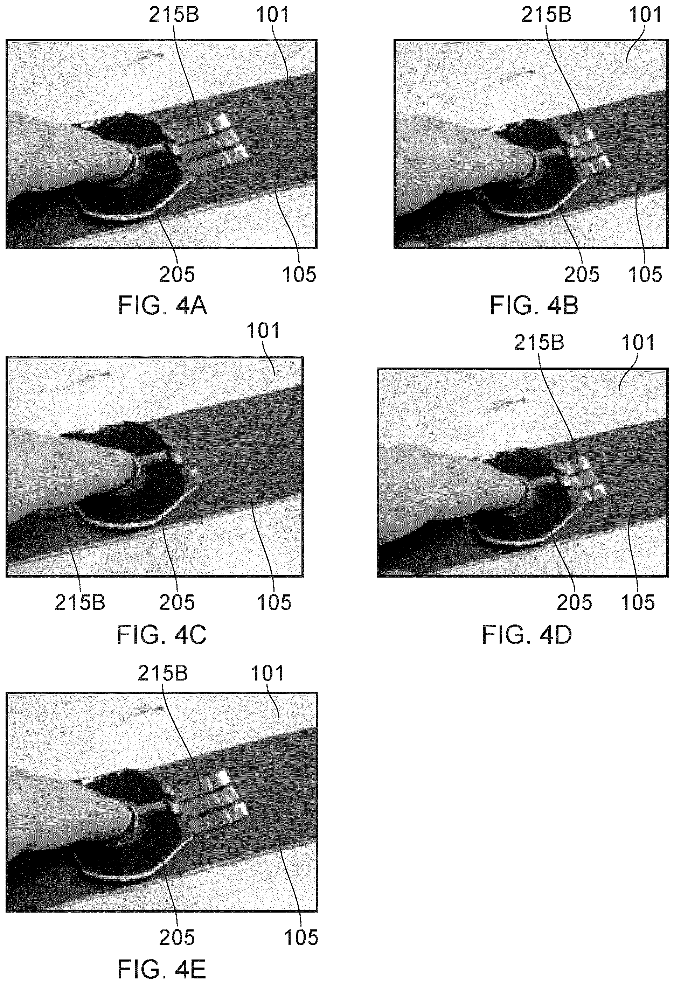

Figs. 4A to 4E are photographs of an example apparatus 101 which show movement of the sensor module 205 relative to the attachment means 105. The apparatus 101 could be the same apparatus 101 as shown in the photographs of Figs. 3A to 3D.

In the photograph of Fig. 4A the sensor module 205 is provided in a first position towards a first end of the second conductive portion 215B. In Fig. 4B the sensor module 205 has been moved towards the right so that the sensor module 205 is now provided in a central position of the second conductive portion 215B. In Fig. 4C the sensor module 205 has been moved further to the right so that the senor module 205 is now provided at the second end of the second conductive portion 215B. The sensor module 205 is now provided at the opposite end of the second conductive portion 215B to the position shown in Fig. 4A. In Fig. 4D the sensor module 205 has been moved back towards the left so that the sensor module 205 is returned to a central position of the second conductive portion 215B. In Fig. 4E the sensor module 205 is returned to the original start position at a first end of the second conductive portion 215B.

In the example of Figs. 4A to 4E the sensor module 205 is moved along the second conductive portion 215B by the force of a finger being applied to the sensor module 205. It is to be appreciated that this is just to show how the sensor module 205 moves relative to the attachment means 105.

In examples of the disclosure the adjustment means may enable movements of the sensor module 205 by several mm while the apparatus 101 is in use. This may enable small relative movements of the apparatus 101 to be accounted for.

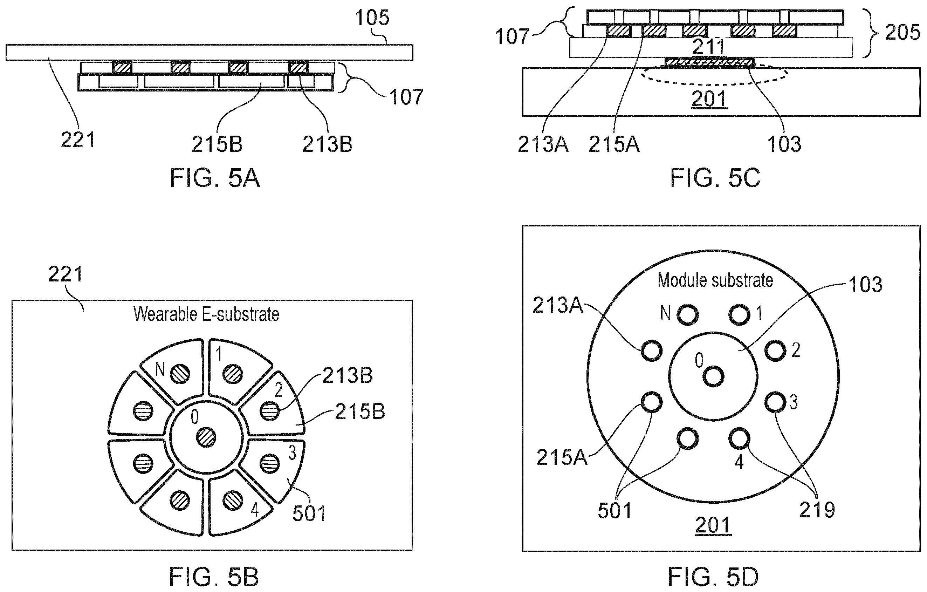

In the examples of Figs 2 to 4E the sensor module 205 has one degree of freedom relative to the attachment means 105. That is, in the example of Figs. 2 to 4E the sensor module 205 can move along the length of the attachment means 105 but not in other directions. Figs. 5A to 5D illustrates another example apparatus 101 in which the sensor module 205 has two or more degrees of freedom of movement. In the example of Figs. 5A and 5D the sensor module 205 may be configured to move both rotationally and laterally compared to the attachment means 105.

Fig. 5A shows a cross section of the attachment means 105 and Fig. 5B shows a plan view of the attachment means 105. Fig. 5C shows a cross section of the sensor modules 205 and Fig. 5D shows a plan view of the sensor module 205. Although the sensor module 205 and the attachment means 105 are shown separately in the examples of Figs. 5A to 5D they would be coupled together when the apparatus 101 is in use.

The sensor 103 is provided within the sensor module 205. The sensor 103 could be an optical sensor or any other suitable type of sensor.

The sensor module 205 comprises a substrate 211. The one or more sensors 103 are mounted on a first surface of the substrate 211. The substrate 211 may be configured to bear the weight, or at least partially, bear the weight of the sensor 103. The substrate 211 may also comprise additional components such as electrical connections which may enable the sensor 103 to be electrically connected to other components of the apparatus 101.

In the example of Figs. 5A to 5D the substrate 211 of the sensor module 205 has a circular or substantially circular shape. The sensor 103 is provided in the centre of the circle. The sensor 103 or sensors 103 could be provided in different positions in other examples of the disclosure.

In the example of Figs. 5A to 5D the substrate 211 also comprises part of the attachment means 107. The parts of the attachment means 107 that are provided on the substrate 211 comprise a first magnetic portion 213A and a first conductive portion 215A. The first magnetic portion 213A and the first conductive portion 215A are provided on an opposing surface of the substrate 211 to the sensors 103.

In the example of Figs. 5A to 5D the first magnetic portion 213A comprises a plurality of magnetic blocks 219. The plurality of magnetic blocks 219 are arranged in a circular configuration. In the example of Fig. 5D the eight magnetic blocks 219 are arranged in a circle. In other examples other numbers of magnetic blocks 219 may be provided.

In the example of Fig. 2 the first conductive portion 215A is also provided on the substrate 211. In the example of Fig. 2 the first conductive portion 215A is provided overlaying the first magnetic portion 213A. The first conductive portion 215A may be provided on the substrate 211 so that the first conductive portion 215A can be in contact with other conductive portions of the apparatus 101. For example the first conductive portion 215A may be in contact with a corresponding second conductive portion 215B provided on the attachment means 105.

In the example of Figs. 5A to 5D the first conductive portion 215A comprises a plurality of conductive pads 501. In the example of Figs. 5A to 5D the conductive pads 501 are arranged in a circle on the substrate 211. In the Figs. 5A to 5D the number of conductive pads 501 is the same as the number of magnetic blocks 219. The conductive pads 501 are configured so that each conductive pad 501 is positioned overlaying a magnetic block 219.

The parts of the adjustment means 107 that are provided on the sensor module 205 may be coupled to corresponding parts of the adjustment means 107 which may be provided on the attachment means 105. In the example of Figs. 5A to 5D the attachment means 105 may comprise a flexible substrate 221. The flexible substrate 221 may be flexible so that the attachment means 105 can be wrapped around a limb or torso or other part of the body of a subject. In the example apparatus 101 of Figs. 5A to 5D the parts of the adjustment means 107 that are provided on the attachment means 105 comprises a second magnetic portion 213B and a second conductive portion 215B.

The second magnetic portion 213B may have the same, or a similar, configuration to the first magnetic portion 213A. In the example of Fig. 2 the second magnetic portion 213B also comprises an array of magnetic blocks 219 arranged in a circular configuration.

The second magnetic portion 213B is positioned within the apparatus 101 so that it can be aligned with the first magnetic portion 213A. When the second magnetic portion 213B is aligned with the first magnetic portion 213A each of the magnetic blocks 219 in the first magnetic portion may be overlaying a magnetic block 219 in the second magnetic portion 213B. This may enable the sensor module 205 to be attached to the attachment means 105.

The second conductive portion 215B has a corresponding configuration to the first conductive portion 213A. In the example of Figs. 5A to 5D the second conductive portion 215B also comprises a plurality of conductive pads 501. In the example of Figs. 5A to 5D the conductive pads 501 are arranged in a circle on the substrate 211. In the Figs. 5A to 5D the number of conductive pads 501 is the same as the number of magnetic blocks 219. The conductive pads 501 are configured so that each conductive pad 501 is positioned overlaying a magnetic block 219.

The conductive pads 501 which form part of the second conductive portion 215B are larger than the conductive pads 501 which form part of the first conductive portion 215B. This enables movement of the sensor module 205 relative to the attachment means but allows the respective conductive pads 501 to remain in contact with each other.

In the example of Figs. 5A to 5D the conductive pads 501 which form part of the first conductive portions 215A have a circular shape while the conductive pads 501 which form part of the second conductive portion 215B have a segment shape. Different shapes of the respective conductive pads 501 can be used in other examples of the disclosure.

The contact between the conductive members of the first conductive portion 215A and the conductive members of the second conductive portion 215B provides a direct current connection between the first conductive portion 215A and the second conductive portion 215B. This therefore provides a direct current connection between the sensor 103 and any other components, such as circuitry or transceivers, which may be provided on the attachment means 105.

In the example of Figs5A to 5D the conductive pads 501 in the second conductive portion 215B have a larger area than the conductive pads 501 in the first conductive portion 215A. This enables the sensor module 205 to be positioned at different positions on the attachment means 105 whilst maintain the electrical connection between the respective conductive portions 215A, 215B. This may enable movement of the sensor module 205 in more than one dimension relative to the attachment means 105 and so enables the position of the sensor module 205 to be adjusted relative to the attachment means 105. When the apparatus 101 is in use and attached to a subject this may enable the sensor module 205 and sensor 103 to be fixed relative to the subject while the attachment means 105 may be moved.

In examples of the disclosure the friction between the sensor module 205 and the subject's skin 201 may help to reduce movement of the sensors 103. In some examples the sensor module 205 may comprise means for increasing the friction between the subject's skin 201 and the sensor module 205. This may act to help keep the sensor module 205 in a fixed position when the attachment means 105 moves. The means for increasing the friction between the subject's skin 201 and the sensor module 205 may comprise a material with a higher coefficient of friction than the material of the substrate 211 of the sensor module. In some examples the means for increasing the friction between the subject's skin 201 and the sensor module 205 may comprise one or more pads which may be provided on the substrate 211 of the sensor module 205. The pads may be positioned such that when the apparatus 101 is attached to a subject the one or more pads contact the subject. The pads may comprise an elastomeric material or any other suitable type of material. Other means may be used in other examples of the disclosure.

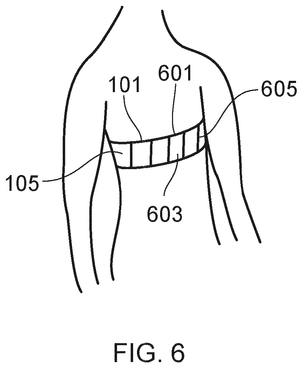

Fig. 6 schematically illustrates a wearable device 601 comprising an example apparatus 101. The apparatus 101 could be as described.

In the example of Fig. 6 the wearable device 601 may also comprise controlling circuitry 603 and one or more transceivers 605. The controlling circuitry 603 and the one or more transceivers 605 may be provided on the attachment means 105. The controlling circuitry 603 and/or the transceiver 605 could be configured to be electrically connected to the one or more sensor 103 via the conductive portions 215A, 215B.

In the example of Fig. 6 the attachment means 105 comprises a chest strap. This may enable the sensor 103 of the apparatus to be positioned adjacent to the chest of the subject. Other types of attachment means may be used in other example wearable devices 601.

The controlling circuitry 603 may comprise means for controlling the apparatus 101. The controlling circuitry 603 may comprise means for controlling the reading of data from and/or the storage of data from the one or more sensors 103.

In some examples the wearable device 601 may also comprise a transceiver 605. The transceiver could be configured to enable data to be transmitted between the wearable device 601 and another device. The another device could be a mobile phone, a communications device or any other suitable type of device.

In some but not necessarily all examples, the controlling circuitry 603 and the transceiver 605 may be configured to communicate data from the wearable device 601 with or without local storage of the data in a memory provided within the wearable device and with or without local processing of the data by circuitry or processors within the wearable device 601.

The data may, for example, be measurement data from the one or more sensors 103. In some examples the data could be data produced by the processing of measurement data from the one or more sensors 103. For example the data could be the heart rate of the subject which is obtained by processing measurements of the light reflected from subject's skin.

The data may be stored in processed or unprocessed format remotely at one or more devices. In some examples the data may be stored in The Cloud.

The data may be processed remotely at one or more devices. The data may be partially processed locally and partially processed remotely at one or more devices.

The data may be communicated to the remote devices wirelessly via short range radio communications such as Wi-Fi or Bluetooth, for example, or over long range cellular radio links. The apparatus may comprise a communications interface such as, for example, a radio transceiver for communication of data.

The wearable device 601 may be part of the Internet of Things forming part of a larger, distributed network.

The processing of the data, whether local or remote, may be for the purpose of health monitoring, data aggregation, patient monitoring, vital signs monitoring or other purposes.

In some examples the processing of the data, whether local or remote, may produce an output. The output may be communicated to the wearable device 601 where it may produce an output which can be sensed by the subject. The output could be an audio output, visual output, haptic output or any other suitable type of output.

It is to be appreciated that variations may be made to some of the apparatus 101 and wearable devices 601 as described. For instance, in some examples the apparatus 101 could comprise additional sensors. The additional sensors could be configured to detect parameters relating to the environment in which the subject is located. For example, the parameters sensed by the additional sensors could comprise the ambient temperature, the presence of humidity or other chemicals, ambient light, electromagnetic radiation levels, noise or any other suitable parameter. These additional sensors may enable information about the environment of the subject to be obtained, in addition to information about the subject itself.

Examples of the disclosure therefore provide for an apparatus 101 which can be attached to a subject. As the sensors 103 of the apparatus can be moved relative to the attachment means 107 of the subject this allows the attachment means to move during use while enabling the sensor 103 to stay in the same position relative to the subject. This enables the sensor to obtain consistent and more reliable data. This may also make the apparatus 101 more comfortable for the subject to wear as he attachment means 107 need to be so tight as to prevent any movement of the attachment means 107.

In the examples describe above the term coupled means operationally coupled. Any number of components may be provided between coupled components including zero components.

The term "comprise" is used in this document with an inclusive not an exclusive meaning. That is any reference to X comprising Y indicates that X may comprise only one Y or may comprise more than one Y. If it is intended to use "comprise" with an exclusive meaning then it will be made clear in the context by referring to "comprising only one..." or by using "consisting".

In this brief description, reference has been made to various examples. The description of features or functions in relation to an example indicates that those features or functions are present in that example. The use of the term "example" or "for example" or "may" in the text denotes, whether explicitly stated or not, that such features or functions are present in at least the described example, whether described as an example or not, and that they can be, but are not necessarily, present in some of or all other examples. Thus "example", "for example" or "may" refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example but does not necessarily have to be used in that other example.

Although embodiments of the present invention have been described in the preceding paragraphs with reference to various examples, it should be appreciated that modifications to the examples given can be made without departing from the scope of the invention as claimed.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

Whilst endeavoring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. An apparatus (101) comprising:
a sensor module (205) comprising one or more sensors (103) wherein the one or more sensors are configured to detect one or more biometric parameters of a subject;
means (105) for attaching the apparatus to a subject; and
means (107) for adjusting the position of the sensor module relative to the means for attaching the apparatus to a subject such that when the apparatus is attached to a subject the sensor module can move relative to the means for attaching the apparatus to a subject, but movement of the sensor module relative to the subject is restricted, the means for adjusting comprising:
a first magnetic portion (213A), provided within the sensor module; and
a second magnetic portion (213B), provided on the means for attaching the apparatus to a subject.

2. An apparatus as claimed in claim 1 wherein the apparatus is configured such that when the apparatus is attached to a subject by the means for attaching the apparatus to a subject, the one or more sensors are positioned adjacent to the subject.

3. An apparatus as claimed in any preceding claim wherein the sensor module comprises one or more pads on a surface of the sensor module wherein the pads are positioned such that when the apparatus is attached to a subject the one or more pads contact the subject.

4. An apparatus as claimed in claim 3 wherein the one or more pads comprise elastomeric material.

5. An apparatus as claimed in any preceding claim wherein the means for adjusting comprises one or more magnetic portions.

6. An apparatus as claimed in claim 6 wherein the means for adjusting comprises an array of magnets arranged in a repelling configuration.

7. An apparatus as claimed in any preceding claim wherein the means for adjusting comprises one or more conductive portions.

8. An apparatus as claimed in any preceding claim wherein the means for adjusting is configured to enable the one or more sensors to move in a longitudinal direction relative to the means for attaching the apparatus to a subject.

9. An apparatus as claimed in any preceding claim wherein the means for adjusting is configured to enable the one or more sensors to rotate relative to the means for attaching the apparatus to a subject.

10. An apparatus as claimed in any preceding claim wherein the means for attaching the apparatus to a subject comprises a strap.

11. An apparatus as claimed in any preceding claim wherein the one or more sensors comprise one or more optical sensors.

12. A wearable electronic device comprising an apparatus as claimed in any preceding claim and controlling circuitry.

13. A wearable electronic device as claimed in claim 12 comprising one or more transceivers.

## Patentansprüche

1. Einrichtung (101), die Folgendes umfasst:
ein Sensormodul (205), das einen oder mehrere Sensoren (103) umfasst, wobei der eine oder die mehreren Sensoren dazu ausgelegt sind, einen oder mehrere biometrische Parameter einer Person zu detektieren;
Mittel (105) zum Befestigen der Einrichtung an einer Person; und
Mittel (107) zum Anpassen der Position des Sensormoduls relativ zu den Mitteln zum Befestigen der Einrichtung an einer Person, derart, dass, wenn die Einrichtung an einer Person befestigt ist, das Sensormodul sich relativ zu den Mitteln zum Befestigen der Einrichtung an einer Person bewegen kann, eine Bewegung des Sensormoduls relativ zur Person aber eingeschränkt ist, wobei die Mittel zum Anpassen Folgendes umfassen:
einen ersten magnetischen Abschnitt (213A), der im Sensormodul bereitgestellt ist; und
einen zweiten magnetischen Abschnitt (213B), der an den Mitteln zum Befestigen der Einrichtung an einer Person bereitgestellt ist.

2. Einrichtung nach Anspruch 1, wobei die Einrichtung derart ausgelegt ist, dass, wenn die Einrichtung mit den Mitteln zum Befestigen der Einrichtung an einer Person an einer Person befestigt ist, der eine oder die mehreren Sensoren neben der Person positioniert sind.

3. Einrichtung nach einem der vorhergehenden Ansprüche, wobei das Sensormodul eine oder mehrere Auflagen auf einer Fläche des Sensormoduls umfasst, wobei die Auflagen derart positioniert sind, dass, wenn die Einrichtung an einer Person befestigt ist, die eine oder die mehreren Auflagen die Person berühren.

4. Einrichtung nach Anspruch 3, wobei die eine oder die mehreren Auflagen ein Elastomermaterial umfassen.

5. Einrichtung nach einem der vorhergehenden Ansprüche, wobei die Mittel zum Anpassen einen oder mehrere magnetische Abschnitte umfassen.

6. Einrichtung nach Anspruch 6, wobei die Mittel zum Anpassen ein Array von Magneten umfassen, die in einer abweisenden Auslegung angeordnet sind.

7. Einrichtung nach einem der vorhergehenden Ansprüche, wobei die Mittel zum Anpassen einen oder mehrere leitende Abschnitte umfassen.

8. Einrichtung nach einem der vorhergehenden Ansprüche, wobei die Mittel zum Anpassen dazu ausgelegt sind, es dem einen oder den mehreren Sensoren zu ermöglichen, sich in einer Längsrichtung relativ zu den Mitteln zum Befestigen der Einrichtung an einer Person zu bewegen.

9. Einrichtung nach einem der vorhergehenden Ansprüche, wobei die Mittel zum Anpassen dazu ausgelegt sind, es dem einen oder den mehreren Sensoren zu ermöglichen, sich relativ zu den Mitteln zum Befestigen der Einrichtung an einer Person zu drehen.

10. Einrichtung nach einem der vorhergehenden Ansprüche, wobei die Mittel zum Befestigen der Einrichtung einer Person einen Gurt umfassen.

11. Einrichtung nach einem der vorhergehenden Ansprüche, wobei der eine oder die mehreren Sensoren einen oder mehrere optische Sensoren umfassen.

12. Tragbare elektronische Vorrichtung, die eine Einrichtung nach einem der vorhergehenden Ansprüche und eine Steuerschaltung umfasst.

13. Tragbare elektronische Vorrichtung nach Anspruch 12, die einen oder mehrere Sendeempfänger umfasst.

## Revendications

1. Appareil (101) comprenant :
un module capteur (205) comprenant un ou plusieurs capteurs, (103) dans lequel les un ou plusieurs capteurs sont configurés pour détecter un ou plusieurs paramètres biométriques d'un sujet ;
des moyens (105) pour fixer l'appareil à un sujet ; et
des moyens (107) pour régler la position du module capteur par rapport aux moyens de fixation de l'appareil à un sujet de sorte que le module capteur puisse se déplacer par rapport aux moyens de fixation de l'appareil à un sujet lorsque l'appareil est fixé à un sujet, mais que le déplacement du module capteur par rapport au sujet soit limité, les moyens de réglage comprenant :
une première partie magnétique (213A) prévue à l'intérieur du module capteur ; et
une deuxième partie magnétique (213B) prévue sur les moyens de fixation de l'appareil à un sujet.

2. Appareil selon la revendication 1, dans lequel l'appareil est configuré de sorte que, lorsque l'appareil est fixé à un sujet par les moyens de fixation de l'appareil à un sujet, les un ou plusieurs capteurs soient positionnés adjacents au sujet.

3. Appareil selon l'une des revendications précédentes, dans lequel le module capteur comprend un ou plusieurs tampons sur une surface du module capteur, dans lequel les tampons sont positionnés de telle sorte que, lorsque l'appareil est fixé à un sujet, les un ou plusieurs tampons entrent en contact avec le sujet.

4. Appareil selon la revendication 3, dans lequel les un ou plusieurs tampons comprennent un matériau élastomère.

5. Appareil selon l'une des revendications précédentes, dans lequel les moyens de réglage comprennent une ou plusieurs parties magnétiques.

6. Appareil selon la revendication 6, dans lequel les moyens de réglage comprennent un réseau d'aimants agencés dans une configuration répulsive.

7. Appareil selon l'une des revendications précédentes, dans lequel les moyens de réglage comprennent une ou plusieurs parties conductrices.

8. Appareil selon l'une des revendications précédentes, dans lequel les moyens de réglage sont configuré pour permettre aux un ou plusieurs capteurs de se déplacer dans une direction longitudinale par rapport aux moyens de fixation de l'appareil à un sujet.

9. Appareil selon l'une des revendications précédentes, dans lequel les moyens de réglage sont configurés pour permettre aux un ou plusieurs capteurs de tourner par rapport aux moyens de fixation de l'appareil à un sujet.

10. Appareil selon l'une des revendications précédentes, dans lequel les moyens de fixation de l'appareil à un sujet comprennent une sangle.

11. Appareil selon l'une des revendications précédentes, dans lequel les un ou plusieurs capteurs comprennent un ou plusieurs capteurs optiques.

12. Dispositif électronique pouvant être porté comprenant un appareil selon l'une des revendications précédentes et une circuiterie de commande.

13. Dispositif électronique pouvant être porté selon la revendication 12 comprenant un ou plusieurs émetteurs-récepteurs.
